# EUROPEAN PATENT APPLICATION

(11) **EP 2 181 660 A1**
(43) Date of publication of application: **05.05.2010**
(21) Application number: 09173784.1
(22) Date of filing: 22.10.2009
(51) Int. Cl.: A61B 17/70

(54) **Spinal implants having multiple movable members**

(30) Priority: 29.10.2008 US 260551
(71) Applicant: Kyphon SÀRL, 2000 Neuchâtel (CH)
(72) Inventor: Kohm, Andrew C., San Mateo, CA 94403 (US); Phan, Christopher U., San Leandro, CA 94578 (US)
(74) Representative: Price, Nigel John King

(57) **Abstract**

An apparatus includes a spinal implant configured to be disposed within an interspinous process space between a first spinous process of a spinal column and a second spinous process of the spinal column. The spinal implant includes a plurality of elongate spacers. A first elongate spacer from the plurality of elongate spacers is configures to slidably contact at least a second elongate spacer from the plurality of elongate spacers. The spinal implant includes an elastic member configured to allow movement of each elongate spacer from the plurality of elongate spacers relative to the remaining elongate spacers from the plurality of elongate spacers within a predetermined range of motion.

## Description

### Background

The disclosed embodiments relate generally to the treatment of spinal conditions including, for example, the treatment of spinal compression using percutaneous spinal implants for implantation between adjacent spinous processes.

A back condition that impacts many individuals is spinal stenosis. Spinal stenosis is a progressive narrowing of the spinal canal that causes compression of the spinal cord and nerve roots extending from the spinal cord. Each vertebra in the spinal column has an opening extending therethrough. The openings are aligned vertically to form the spinal canal, within which the spinal cord is disposed. As the spinal canal narrows from spinal stenosis, the spinal cord and nerve roots extending from the spinal cord and between adjacent vertebrae are compressed and may become inflamed. Spinal stenosis can cause pain, weakness, numbness, burning sensations, tingling, and in particularly severe cases, may cause loss of bladder or bowel function, or paralysis. The legs, calves and buttocks are most commonly affected by spinal stenosis, however, the shoulders and arms may also be affected.

Mild cases of spinal stenosis may be treated with rest or restricted activity, non-steroidal anti-inflammatory drugs (e.g., aspirin), corticosteroid injections (epidural steroids), and /or physical therapy. Some patients find that bending forward, sitting or lying down may help relieve the pain. This may be due to the fact that bending forward results in more vertebral space, which may temporarily relieve nerve compression. Because spinal stenosis is a progressive disease, the source of pressure may be surgically corrected (e.g., via a decompressive laminectomy) as the patient has increasing pain. In such a surgical procedure, bone and other tissues that have impinged upon the spinal canal and/or put pressure on the spinal cord can be removed. Alternatively, two adjacent vertebrae may be fused during the surgical procedure to prevent an area of instability, improper alignment or slippage, such as that caused by spondylolisthesis. In yet another surgical procedure, surgical decompression can relieve pressure on the spinal cord or spinal nerve by widening the spinal canal to create more space. In this procedure, the patient is given a general anesthesia as an incision is made in the patient to access the spine to remove the areas that are contributing to the pressure. This procedure, however, may result in blood loss and an increased chance of significant complications, and usually results in an extended hospital stay.

Thus, a need exists for improvements in spinal implants for implantation between adjacent spinous processes to improve treatment of spinal conditions, such as spinal stenosis.

### Summary of the Invention

Spinal implants and methods are described herein. In some embodiments, an apparatus includes a spinal implant configured to be disposed within an interspinous process space between a first spinous process of a spinal column and a second spinous process of the spinal column. The spinal implant includes multiple elongate spacers. A first elongate spacer from the elongate spacers slidably contacts at least a second elongate spacer from the elongate spacers. The spinal implant includes an elastic member configured to allow movement of each elongate spacer from the elongate spacers relative to the remaining elongate spacers from the elongate spacers within a predetermined range of motion. In some embodiments, for example, the first elongate spacer from the spacers is longitudinally aligned with the second elongate spacer from the spacers. In other embodiments, for example, the elastic member is coupled to at least two elongate spacers from the spacers.

### Brief Description of the Drawings

FIG. 1 is a schematic illustration of a posterior view of a spinal implant according to an embodiment disposed between a first spinous process and a second spinous process.

FIG. 2 is a cross-sectional posterior view of the spinal implant of FIG. 1.

FIG. 3 is a schematic illustration of a side perspective view of the spinal implant of FIG. 1.

FIGS. 4 and 5 are schematic illustrations of lateral views of the spinal implant of FIG. 1 in a first configuration and a second configuration, respectively.

FIG. 6 is schematic illustration of a posterior view of a spinal implant according to an embodiment disposed between a first spinous process and a second spinous process.

FIG. 7 is a top view of the spinal implant of FIG. 6.

FIG. 8 is a cross-sectional view along line D-D of the spinal implant of FIG. 7.

FIG. 9 is a cross-sectional view along line C-C of the spinal implant of FIG. 6.

FIG. 10 is a cross-sectional view along line E-E of the spinal implant of FIG. 7.

FIGS. 11 and 12 are schematic illustrations of lateral views of the spinal implant of FIG. 6 in cross-section in a first configuration and a second configuration, respectively.

FIG. 13 is a schematic illustration of a spinal implant according to an embodiment.

FIG. 14 is a schematic illustration of a spinal implant according to an embodiment.

FIG. 15 is a perspective view of a portion of the spinal implant of FIG. 14.

FIG. 16 is top view of the spinal implant of FIG. 14.

FIG. 17 is a cross-sectional view along line C₁-C₁ of the spinal implant of FIG. 14.

FIG. 18 is schematic illustrations of a posterior view of a spinal implant according to an embodiment disposed between a first spinous process and a second spinous process.

FIGS. 19 and 20 are schematic illustrations of the implantation of the spinal implant of FIG. 18 between a first spinous process and a second spinous process.

FIG. 21 is a schematic illustration of a posterior view of a spinal implant according to an embodiment disposed between a first spinous process and a second spinous process.

FIG. 22 is a schematic illustration of a lateral view of an implant according to an embodiment.

FIG. 23 is a schematic illustration of a posterior view of an implant in a first configuration according to an embodiment.

FIG. 24 is a schematic illustration of the implant of FIG. 23 in a second configuration disposed between a first spinous process and a second spinous process.

FIG. 25 is perspective view of the implant of FIG. 23.

FIG. 26 is a flowchart of a method according to an embodiment.

FIG. 27 is a flowchart of a method according to an embodiment.

### Detailed Description

In some embodiments, an apparatus includes a spinal implant configured to be disposed within an interspinous process space between a first spinous process of a spinal column and a second spinous process of the spinal column. The spinal implant includes a set of elongate spacers and an elastic member. A first elongate spacer from the set of elongate spacers is in slidable contact with at least a second elongate spacer from the set of elongate spacers. The elastic member is configured to allow movement of each elongate spacer from the set of elongate spacers relative to the remaining elongate spacers from the set of elongate spacers within a predetermined range of motion. In some embodiments, for example, the set of elongate spacers collectively has a first shape during flexion of the spinal column and has a second shape, different from the first shape, during extension of the spinal column. In some embodiments, for example, the set of elongate spacers is configured to collectively form a shape substantially corresponding to a shape of at least a portion of the interspinous process space when the spinal implant is disposed within the interspinous process space. In some embodiments, for example, each spacer from the set of spacers is substantially cylindrical and the first elongate spacer is longitudinally aligned with the remaining elongate spacers from the set of elongate spacers. In some embodiments, for example, the elastic member substantially surrounds a perimeter of the set of elongate spacers.

As used in this specification and the appended claims, the singular forms "a," "an" and "the" include plural referents unless the context clearly dictates otherwise. Thus, for example, the term "a member" is intended to mean a single member or a combination of members, "a material" is intended to mean one or more materials, or a combination thereof. Furthermore, the words "proximal" and "distal" refer to direction closer to and away from, respectively, an operator (e.g., surgeon, physician, nurse, technician, etc.) who would insert the medical device into the patient, with the tip-end (i.e., distal end) of the device inserted inside a patient's body first. Thus, for example, the implant end first inserted inside the patient's body would be the distal end of the implant, while the implant end to last enter the patient's body would be the proximal end of the implant.

The term "body" is used here to mean a mammalian body. For example, a body can be a patient's body, or a cadaver, or a portion of a patient's body or a portion of a cadaver or model of a body or model of a portion of a body.

The term "parallel" or is used herein to describe a relationship between two geometric constructions (e.g., two lines, two planes, a line and a plane, two curved surfaces, a line and a curved surface or the like) in which the two geometric constructions are substantially non-intersecting as they extend substantially to infinity. For example, as used herein, a line is said to be parallel to a curved surface when the line and the curved surface do not intersect as they extend to infinity. Similarly, when a planar surface (i.e., a two-dimensional surface) is said to be parallel to a line, every point along the line is spaced apart from the nearest portion of the surface by a substantially equal distance. Two geometric constructions are described herein as being "parallel" or "substantially parallel" to each other when they are nominally parallel to each other, such as for example, when they are parallel to each other within a tolerance. Such tolerances can include, for example, manufacturing tolerances, measurement tolerances or the like.

The terms "perpendicular", "orthogonal", and/or "normal" are used herein to describe a relationship between two geometric constructions (e.g., two lines, two planes, a line and a plane, two curved surfaces, a line and a curved surface or the like) in which the two geometric constructions intersect at an angle of approximately 90 degrees within at least one plane. For example, as used herein, a line is said to be normal to a curved surface when the line and the curved surface intersect at an angle of approximately 90 degrees within a plane. Two geometric constructions are described herein as being, for example, "perpendicular" or "substantially perpendicular" to each other when they are nominally perpendicular to each other, such as for example, when they are perpendicular to each other within a tolerance. Such tolerances can include, for example, manufacturing tolerances, measurement tolerances or the like.

It should be understood that the references to geometric constructions are for purposes of discussion and illustration. The actual structures may differ from geometric ideal due to tolerances and/or other minor deviations from the geometric ideal.

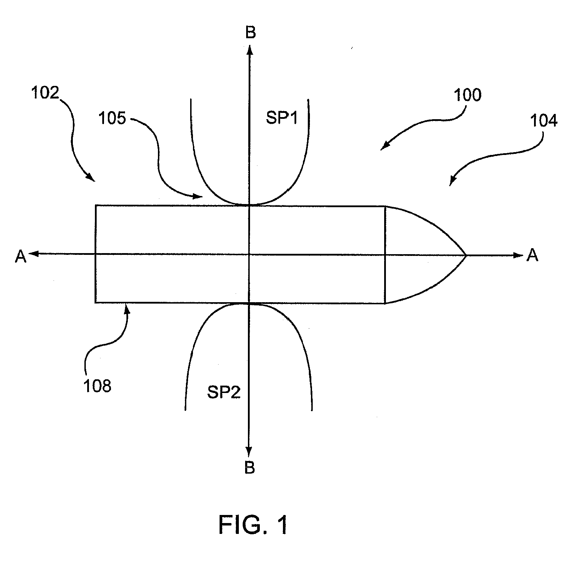

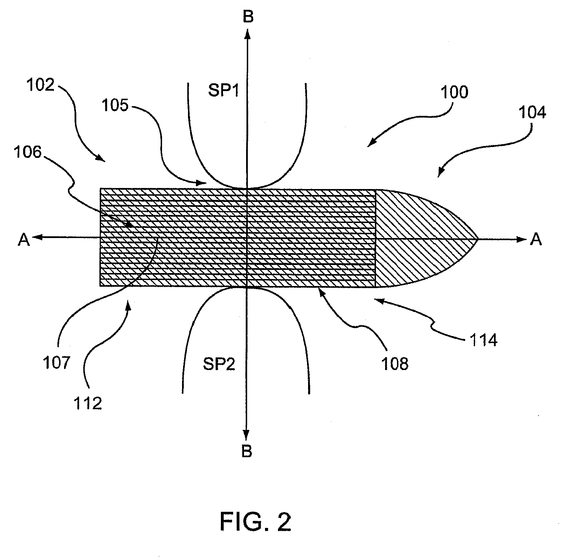

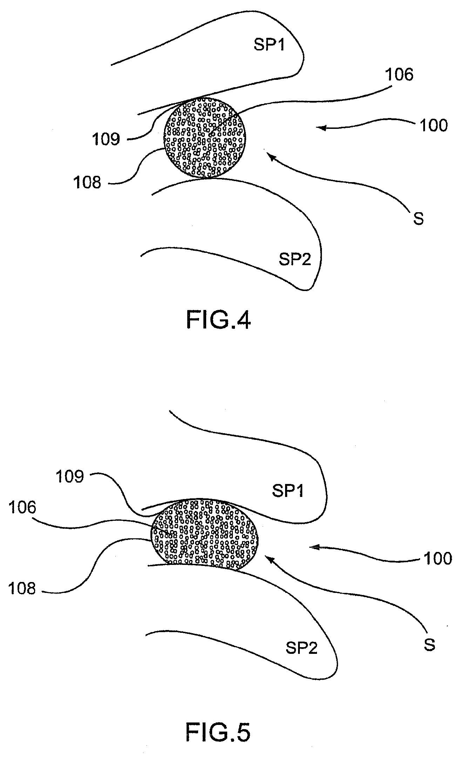

FIGS. 1-5 are schematic illustrations of a spinal implant 100 according to an embodiment. The implant 100 includes a proximal end portion 102, a distal end portion 104, and a middle portion 105. The middle portion 105 includes a set of elongate spacers 106 and an elastic member 108. The implant 100 includes a first configuration (e.g., first shape), shown in FIG. 4, and a second configuration (e.g., second shape), shown in FIG. 5. At least a portion of the implant 100 is configured to be disposed in an interspinous process space S between a first spinous process SP1 and a second spinous process SP2 such that the middle portion 105 of the implant 100 engages the first spinous process SP1 and the second spinous process SP2 during at least spinal extension, either directly or through surrounding tissue. For purposes of clarity, the tissue surrounding the spinous processes SP1, SP2 is not illustrated. The implant 100 is configured to be disposed between the first and second spinous processes SP1, SP2 such that a longitudinal axis A-A defined by a first spacer 107 from the spacers 106 is substantially orthogonal to a mid-line axis B-B defined by the first and second spinous process SP1, SP2. Said another way, the longitudinal axis A-A extends lengthwise (or longitudinally) (e.g., from the proximal end portion 102 to the distal end portion 104) through the center of the first spacer 107 and is configured to be substantially perpendicular with respect to the mid-line axis B-B of the spinous processes SP1, SP2 when the implant 100 is disposed within the interspinous process space S.

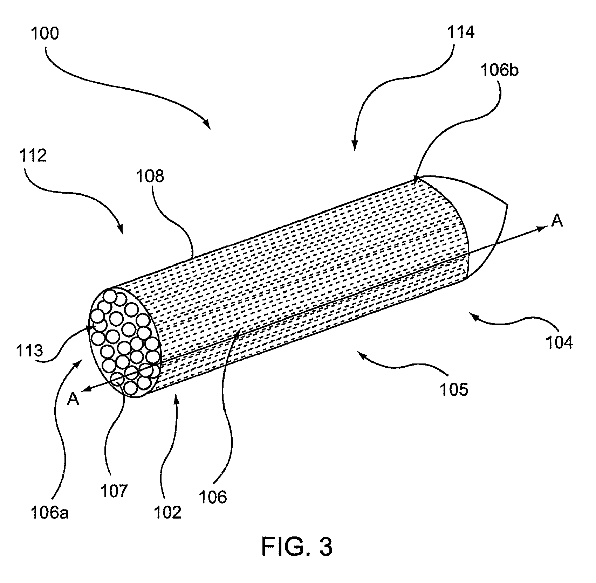

As illustrated in FIG. 3, each spacer from the spacers 106 includes a proximal end portion 106a and a distal end portion 106b. Each spacer from the spacers 106 is substantially cylindrical, having a diameter substantially equal to an adjacent spacer. Each spacer from the spacers 106 is longitudinally aligned with respect to the remaining elongate spacers. Said another way, the longitudinal axis A-A defined by the first spacer 107 from the spacers 106 is substantially parallel to a longitudinal axis defined by the remaining spacers from the spacers 106. Each spacer from the spacers 106 is configured to slidably contact an adjacent spacer 106, and is configured to be independently moveable with respect to an adjacent spacer 106 within a predetermined limited range of motion (described in more detail below).

The elastic member 108 includes a proximal end portion 112, a distal end portion 114, and defines a lumen 113 therethrough. The elastic member 108 is configured to receive the plurality of spacers 106 within the lumen 113 such that each spacer from the spacers 106 is slidably moveable with respect to the remaining spacers 106. Specifically, the elastic member 108 substantially surrounds an outer perimeter of the set of spacers 106, when the set of spacer 106 is disposed lengthwise within the lumen 113 of the elastic member 108. The elastic member 108 is configured to bias the spacers 106, collectively, in a substantially cylindrical configuration when a minimal external load is applied (e.g., during flexion of spinal column), shown, for example in FIG. 4. The elastic member 108, can be, for example, a flexible sheath.

In the illustrated embodiment, at least during spinal extension (FIG. 5), a side wall 109 of the elastic member 108 is configured to directly engage and/or contact the spinous processes SP1, SP2 and/or the bodily tissue surrounding the spinous processes SP1, SP2 without any intervening structure associated with the implant 100. As shown in FIGS. 4 and 5, respectively, the implant 100 is movable between the first configuration (e.g., having a first shape), and the second configuration (e.g., having a second shape). Specifically, the elongate spacers 106 disposed within the elastic member 108 collectively have a first shape during flexion of the spinal column and collectively have a second shape, different from the first shape, during extension of the spinal column. This allows the implant 100 to substantially conform in shape to a shape of at least a portion of the interspinous process space S between spinous processes SP1, SP2. Said another way, during flexion of the spinal column, the spacers 106 collectively are biased to a cylindrical configuration (e.g., having a first shape), shown in FIG. 4. During extension of the spinal column (i.e., when a load is applied), the elastic member 108 surrounding the spacers 106 allows movement of each spacer 106 (e.g., sliding, shifting, rotation, etc.) with respect to the remaining spacers within a range of motion, as shown in FIG. 5. Thus, during extension, the spacers 106 collectively and the side wall 109 of the elastic member 108, conform to portions of the anatomical geometry of the spinous processes SP1, SP2. Similarly stated, during extension the spacers 106 collectively change shape to increase the area of contact between the implant 100 and the spinous processes SP1, SP2.

Although the implant 100 is illustrated and described above as including a set of spacers 106, each having substantially cylindrical configurations and having substantially equal diameters, it should be understood that the spacers 106 can define a variety of shapes, sizes, and configurations. For example, in some embodiments, each spacer can have a substantially spherical configuration. In other embodiments, each spacer can have a substantially rectangular cross section (discussed in more detail herein). In yet other embodiments, each spacer has a diameter and/or size different from a diameter and/or size of an adjacent spacer. Each spacer from the spacers 106 can further be constructed of any suitable material. For example, in some embodiments, the spacers can be constructed of a substantially rigid and/or corrosion-resistant material such as Titanium or Polyetheretherketone (PEEK). The set spacers 106 of implant 100 can also include any suitable number of spacers. For example, in some embodiments, the implant 100 can include two, five, ten, or more spacers.

Although the implant 100 is described above as including an elastic member 108 surrounding the spacers 106, it should be understood that the elastic member 108 can define a variety of shapes, sizes, and/or configurations. For example, in some embodiments the elastic member can be configured to couple the spacers 106 in a longitudinal configuration (discussed in more detail herein). In some embodiments, the elastic member can be configured to extend through each spacer from the spacers 106. In yet other embodiments, an implant can include multiple elastic members.

The elastic member 108 can be constructed of any suitable material for insertion into a body of a patient. For example, in some embodiments, the elastic member can be constructed of a biocompatible silicone or elastomer (e.g., synthetically produced butyl rubber or neoprene or a natural rubber).

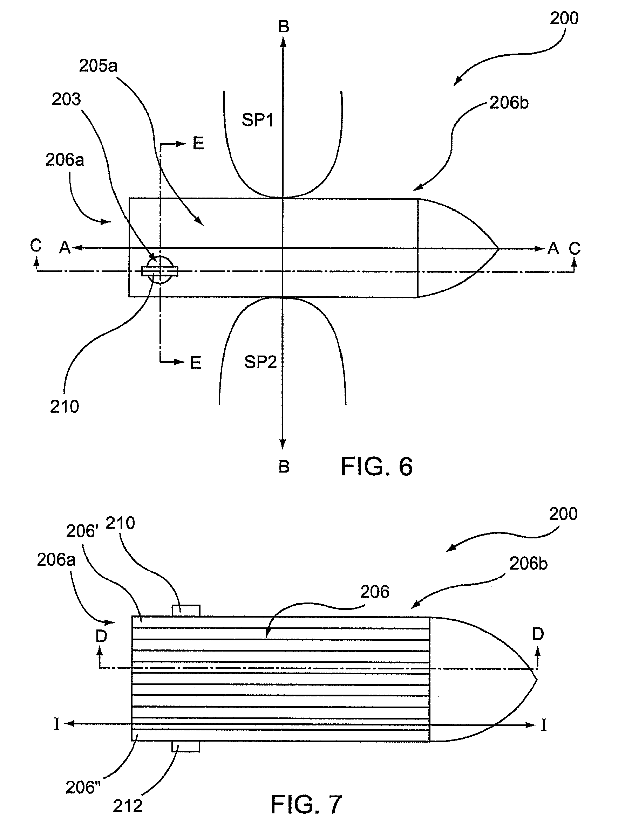

FIGS. 6-12 are schematic illustrations of an implant 200 including a set of spacers 206 according to an embodiment. Each spacer from the set of spacers 206 includes a proximal end portion 206a, a distal end portion 206b, a first surface 205a, a second surface 205b, and defines an opening 203 between the first surface 205a and the second surface 205b. Each spacer from the set of spacers 206 has a substantially rectangular cross section and defines a longitudinal axis I-I (only one such axis is shown in FIG. 7 for purposes of clarity). Each spacer from the set of spacers 206 is longitudinally aligned with respect to the remaining spacers from the spacers 206, and is configured to slidably contact an adjacent spacer from the set of spacers 206. Specifically, the second surface 205b of at least one spacer from the spacers 206 is configured to contact and be slidably moveable with respect to the first surface 205a of an adjacent spacer from the spacers 206.

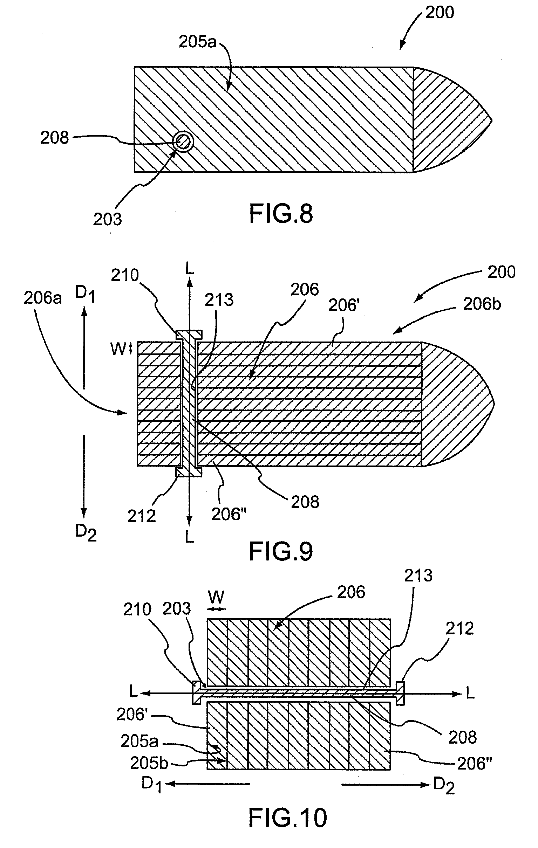

As shown in FIG. 9, the opening 203 extends laterally, from the first surface 205a to the second surface 205b through a width W of each spacer. The opening 203 of each spacer is configured such that at least a portion of the opening 203 is in continuous communication with the opening of an adjacent spacer.

The elastic member 208 includes a first end portion 210, a second end portion 212, a middle portion 213, and defines a longitudinal axis L-L. The elastic member 208 is coupled to at least two adjacent spacers 206, shown, for example in FIGS. 9 and 10. Specifically, the elastic member 208 is slidably disposed within the openings 203 defined by each spacer 206 such that the longitudinal axis L-L defined by the elastic member 208 is substantially perpendicular to the longitudinal axis A-A defined by each spacer 206. In the illustrated embodiment, the first end portion 210 extends outside of the opening 203 of a first outer spacer 206', the second end portion 212 extends outside of the opening 203 of a second outer spacer 206", and the middle portion 213 is disposed within the openings defined by and along the width W of each remaining spacer 206.

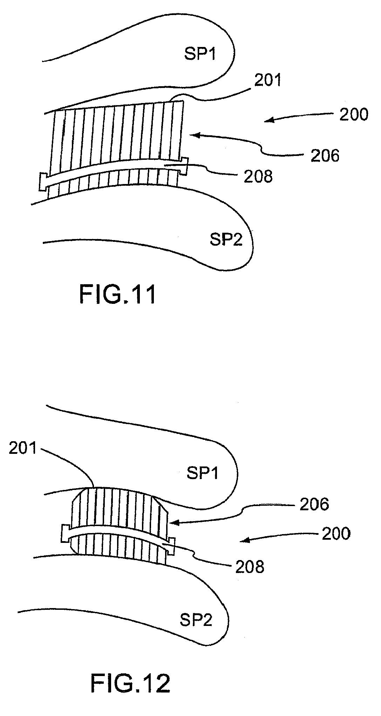

The first end portion 210 of the elastic member 208 is configured to prevent lateral movement of the spacers 206 in a first direction D₁. The second end portion 212 of the elastic member 208 is configured to prevent lateral movement of the spacers 206 in a second direction D₂ opposite the first direction D₁. The middle portion 213 of the elastic member 208 is configured to allow limited movement of the plurality of spacers 206 in a direction substantially perpendicular to the orientation of the elastic member 208 (i.e., substantially perpendicular to the longitudinal axis L-L). Said another way, the middle portion 213 allows limited movement of the spacers 206 in a direction substantially parallel to the longitudinal axis A-A defined by each spacer 206. The elastic member 208 is configured to allow limited movement of each spacer 206 relative to the remaining spacers 206 such that the implant 200 has a first configuration during flexion (shown in FIG. 11) and a second configuration during extension, thus substantially conforming to the anatomical geometry associated with the spinous processes SP1, SP2, shown, for example in FIG. 12.

Referring to FIGS. 11 and 12, at least during spinal extension, the collective outer edge 201 of the spacers 206 is configured to directly engage and/or contact the spinous processes SP1, SP2 and/or the bodily tissue surrounding the spinous processes SP1, SP2 without any intervening structure associated with the implant 200. Moreover, the implant 200 is movable between the first configuration (e.g., having a first shape) and a second configuration (e.g., having a second shape). Said another way, the spacers 206 collectively have a first shape during flexion of the spinal column and collectively have a second shape, different from the first shape, during extension of the spinal column. This allows the implant 200 to substantially conform in shape to a shape corresponding to a shape of at least a portion of the interspinous process space between spinous processes SP1, SP2. Specifically, during extension and flexion of the spinal column, each spacer 206 is configured to move (e.g., slide or shift) with respect to at least one adjacent spacer from the spacers 206. The elastic member 208 allows movement between adjacent spacers 206 such that at least a portion of the collective outer edge 201 of the spacers 206 makes substantially continuous contact with spinous processes SP1 and SP2. Thus, the shape of the spacers 206 substantially conforms to the anatomical geometry of the spinous processes SP1, SP2 as the spinal column undergoes extension.

Each spacer from the set of spacers 206 can be constructed of any suitable material. For example, in some embodiments, the spacers can be constructed of a substantially rigid and/or corrosion-resistant material such as Titanium or Polyetheretherketone (PEEK). In other embodiments, each spacer from the set of spacers 206 can be constructed of a substantially deformable material, which can provide the patient with additional comfort. The deformable material can be, for example a bio-compatible silicone or elastomer (e.g., synthetically produced butyl rubber or neoprene or a natural rubber).

Although the implant 200 is described above as including the elastic member 208 being disposed within openings 203 of each spacer, it should be understood that the elastic member 208 can couple each spacer by any suitable means. For example, in some embodiments, the elastic member 208 can be disposed about a collective outer surface of the spacers 206. In other embodiments, multiple elastic members are disposed about an outer surface of the spacers 206. In yet other embodiments, an elastic member from the elastic members can be disposed between each spacer from the multiple spacers. Said another way, an elastic member can be disposed along a second surface of at least a first elongate member and along a first surface of an adjacent second elongate member.

The elastic member 208 can be constructed of any suitable material for insertion into a body of a patient. For example, in some embodiments, the elastic member can be constructed of a bio-compatible silicone or elastomer (e.g., synthetically produced butyl rubber or neoprene or a natural rubber).

Although the opening 203 defined by each spacer 206 is illustrated as being a circular shape, any suitable shaped opening can be defined by each spacer. For example, the opening can be rectangular shaped, square shaped, triangular shaped, etc.

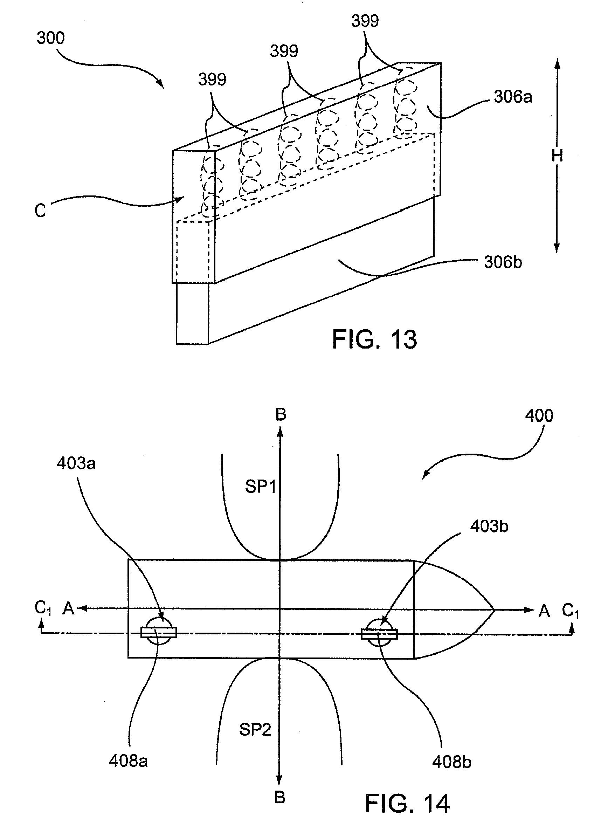

Although the implant 200 is illustrated and described above as including multiple spacers 206, each having a substantially solid rectangular cross section, it should be understood that the spacers 206 can define a variety of shapes, sizes, and configurations. The multiple spacers 206 can also include any suitable number of spacers. For example, in some embodiments, such as the embodiment partially shown in FIG. 13, each spacer 300 includes an outer portion 306a and an inner portion 306b where the outer portion 306a defines a cavity C and is configured to slidably receive the inner portion 306b. In such an embodiment, each spacer 306 has a first configuration (i.e., collapsed configuration) and a second configuration (i.e., expanded configuration). During extension of the spinal column, each spacer 306 is in its first configuration (i.e., collapsed configuration) where the outer portion 306a substantially receives the inner portion 306b. During flexion of the spinal column, each spacer 306 is in its second configuration (i.e., expanded configuration) wherein the outer spacer 306a receives only a portion of the inner spacer 306b. Each spacer 306 is configured to be biased to its expanded configuration and is moveable to its collapsed configuration when a load is applied (e.g., during extension of the spinal column). Said another way, a height H of each spacer 306 (e.g., inner portion and outer portion, collectively) is variable depending upon the load applied. Each spacer 306 can be biased by any suitable biasing mechanism. For example, as shown in FIG. 13, each spacer 306 can be biased by springs 399.

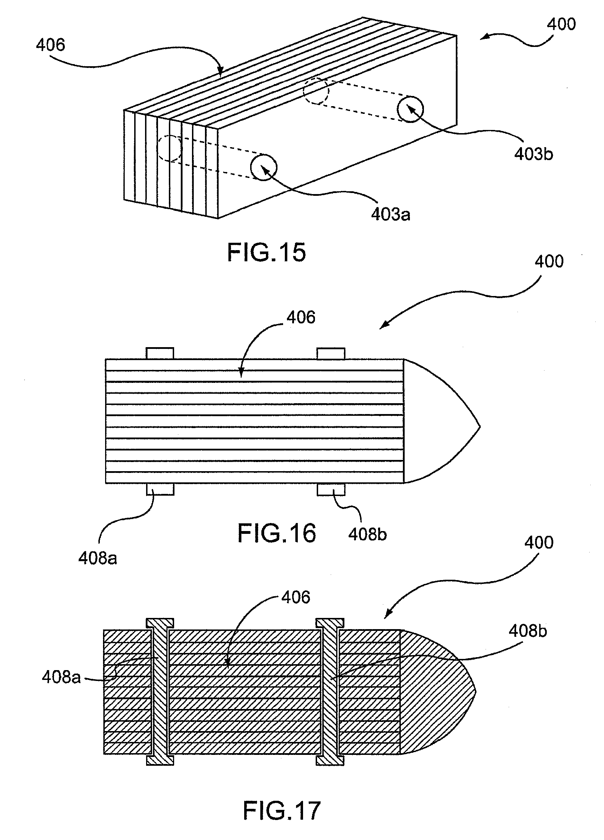

Although the implant 200 is described above as having one elastic member 208, multiple elastic members can be used to slidably couple the spacers 206. For example, in some embodiments, a suitable number of elastic members can include two, three four, or more elastic members. In one such embodiment, as shown in FIGS. 14-17, each spacer from the set of spacers 406 includes multiple openings 403a, 403b. The openings 403a, 403b are configured to receive at elastic members 408a, 408b, respectively, as shown in FIG. 14-17. Each spacer can define any suitable number of openings. For example, each spacer can include one, two, three or more openings.

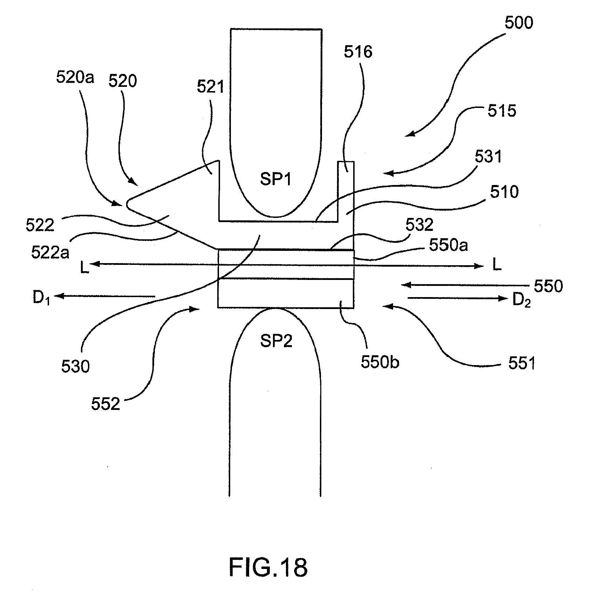

FIGS. 18-20 are schematic illustrations of an implant 500 according to another embodiment. FIGS. 18-20 illustrate posterior views of the implant 500 disposed between a first spinous process SP1 and a second spinous process SP2 adjacent the first spinous process SP1. The implant 500 includes a spacer 510 and one or more elongate members 550 configured to be slidably coupled to the spacer 510. Although FIGS. 18-20 show two elongate members 550, in other embodiments, the implant 500 can include any suitable number of elongate members 550. As described in greater detail below, during implantation of the implant 500, the spacer 510 and the elongate members 550 are separately and/or serially inserted and/or positioned within the body.

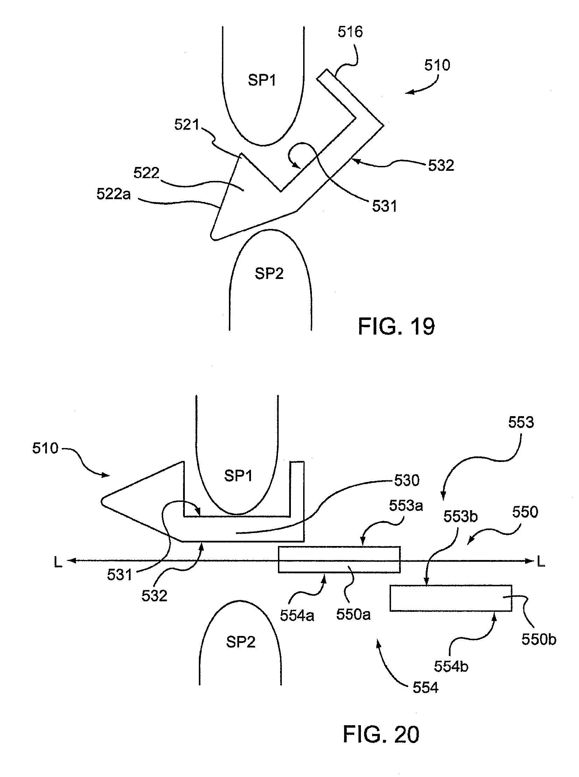

The spacer 510 includes a proximal end portion 515, a distal end portion 520, and a support portion 530. The proximal end portion 515 includes a first retention portion 516 configured to limit lateral movement of the spacer 510 in a first direction D₁. The distal end portion 520 includes a second retention portion 521 configured to limit lateral movement of the spacer 510 in a second direction D₂ opposite the first direction D₁. The support portion 530 includes a first surface 531 and a second surface 532, and extends between the first retention portion 516 and the second retention portion 521. Thus, the spacer 510 defines a saddle shape such that when the spacer 510 is positioned between adjacent spinous processes, the first surface 531 of the support portion 530 engages the first spinous process SP1, the first retention portion 516 is disposed on a first side of a first spinous process SP1, and the second retention portion 521 is disposed on a second side of the first spinous process SP2. Said another way, the first retention portion 516 and the second retention portion 521 of the spacer 510 are collectively configured to receive a portion of a first spinous process SP1, as shown, for example in FIGS. 18 and 20.

The distal end portion 520 includes a distraction portion 522 to assist in the insertion of the spacer 510 between the first spinous process SP1 and the second spinous process SP2. The distraction portion 522 defines a contact surface 522a. The contact surface 522a is tapered to a distal end 520a of the spacer 510. Said another way, the width of the distraction portion 522 substantially continuously decreases towards the distal end 520a of the spacer 510. The tapered configuration facilitates implantation of the spacer 510 between the first and second spinous processes SP1, SP2, as shown in FIG. 19.

Each elongate member 550 from the set elongate members 550 includes a proximal end portion 551, a distal end portion 552, an upper surface 553, and a lower surface 554. Each elongate member from the set elongate members 550 defines a longitudinal axis L-L (only one such axis is shown in FIG. 18 for purposes of clarity). Each elongate member 550 is longitudinally aligned with respect to the remaining elongate members from the set of elongate members 550 and is configured to slidably engage an adjacent elongate member 550. Specifically, as shown in FIG. 20, the lower surface 554a of at least one elongate member 550a from the set of elongate member 550 is configured to contact and/or slidably engage the upper surface 553b of an adjacent elongate member 550b from the set of elongate members 550 (discussed in more detail below). Moreover, an upper surface 553a of a first elongate member 550a from the elongate members 550 is configured to slidably engage the second surface 532 of the spacer 510 when the spacer 510 is positioned between the first and second spinous processes SP1, SP2.

The distal end portion 552 of the elongate members 550 can have any suitable configuration. For example, in some embodiments, the distal end portion can be tapered or beveled to facilitate insertion of the elongate members with respect to the spacer and the remaining elongate members. In other embodiments, the distal end portion can be squared. In such embodiments, an insertion tool (not illustrated) can be used to lift the proximal end portion of the spacer and slide the elongate members such that the upper sliding surface of the elongate member is slidably coupled to the lower surface of the support portion of the spacer. The insertion tool can be, for example, a cannula having an opening at its distal end and a tapered distal and portion leading to the opening. Specifically, in use, the distal end portion of the insertion tool is inserted between the spacer and the second spinous process. An elongate member from the elongate members is inserted into the lumen of the cannula. The elongate member is advanced through the lumen and out the opening at the distal end of the cannula. The remaining elongate members can be inserted in a similar manner creating a stacked configuration.

The upper sliding surface 553 of the elongate members 550 can have any suitable surface configuration to provide various degrees of freedom to accommodate various surgical procedures. For example, in some embodiments, the upper surface 553 can be substantially smooth (i.e., substantially devoid of surface texture) allowing the elongate members to easily move relative to the spacer and the remaining elongate members. In other embodiments, the upper surface 553 can be textured.

The first elongate member 550a from the elongate members 550 can be slidably engaged to the spacer 510 by any suitable mechanism for providing various degrees of freedom. For example, in some embodiments the first elongate member 550a and spacer 510 are slidably coupled. For example, in some embodiments, the first elongate member 553a can be magnetically coupled to spacer 510. In other embodiments, the first elongate member 550a and the spacer 510 can include complimentary projections and/or detents. Specifically, the upper surface 553a of the first elongate member 550a can include at least one projection. In such embodiments, the second surface 532 of the support portion of the spacer 510 includes at least one detent corresponding to the at least one projection. In yet other embodiments, for example, the upper surface 553a of the first elongate member 550a can include at least one ridge extending from the proximal end portion 551 to the distal end portion 552 of the first elongate member 550a. In such embodiments, the lower surface 532 of the support portion of the spacer 510 includes at least one groove corresponding to the at least one ridge.

The upper surfaces 553 of the remaining elongate members 550 can be slidably engaged to the lower surfaces of adjacent elongate members 550 by any of the suitable mechanism for providing various degrees of freedom as discussed above with respect to the first elongate member 550a and the spacer 510. For example, in some embodiments, the upper surface 553b of the second elongate spacer 550b from the elongate spacers 550 can be magnetically coupled to a lower surface 554a of the first elongate member 550a. In other embodiments, the upper surface 553b of the second elongate member 550b can include at least one ridge extending from the proximal end portion 551 to the distal end portion 552 of the second elongate member. In such embodiments, the lower surface 554a of the first elongate member 550a includes at least one groove corresponding to the at least one ridge. In yet other embodiments, the upper surface 553b of the second elongate member 550b and the lower surface 554a of the first elongate member 550a can include mating protrusions and/or openings to releasably and slidably couple the second elongate member 550b to the first elongate member 550a.

Each elongate member from the elongate members 550 can be constructed of any suitable material. For example, in some embodiments, the elongate members can be constructed of a substantially rigid and/or corrosion-resistant material such as Titanium or Polyetheretherketone (PEEK). In other embodiments, the elongate members can be constructed of a substantially flexible material.

The elongate members 550 of implant 500 can also include any suitable number of elongate members 550 for providing an appropriate and/or desired amount of distraction between the implant 500 and spinous processes SP1 and SP2. For example, in some embodiments, the implant can include two, three, four, or more spacers (as shown, for example, in FIGS. 18, 20-22)

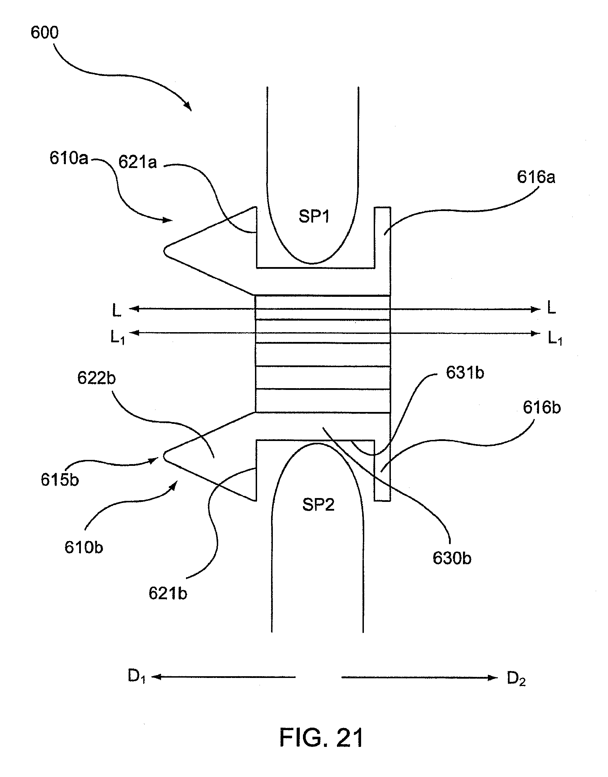

Although the implant 500 is described above as including one spacer 510, it should be understood that other configurations are possible. For example, in some embodiments, as shown in FIG. 21, the implant 600 can include a second spacer 610b, in addition to a first spacer 610a. The first spacer 610a is similar in structure and function to the spacer 510, and thus is not described in detail below. In such an embodiment, the first retention portion 616b of the second spacer 610b is configured to limit lateral movement of the second spacer 610b in a first direction D₁. The second retention portion 621b is configured to limit lateral movement of the second spacer 610b in a second direction D₂ opposite the first direction D₁. The distal end portion 615b of the second spacer 610b further includes a distraction element 622b to assist in the insertion of the second spacer 610b between the first spinous process SP1 and the second spinous process SP2. The second spacer 610b defines a saddle shape, such that when the second spacer 610b is positioned between adjacent spinous processes, the first surface 631b of the support portion 630b engages the second spinous process SP2, the first retention portion 616b is disposed on a first side of a second spinous process SP2, and the second retention portion 621b is disposed on a second side of the second spinous process SP2. Said another way, the first retention portion 616b and the second retention portion 621b of the second spacer 610b are configured to receive a portion of the second spinous process SP2, as shown, for example in FIG. 21.

In use, the first spacer 610a is inserted between the first spinous process SP1 and the second spinous process SP2 such that a first retention portion 616a and the second retention portion 621a of the first spacer 610a are configured to receive a portion of the first spinous process SP2, The second spacer 610b is then inserted between the first spacer 610a and the second spinous process SP2 such that the first retention portion 616b and the second retention portion 621 b of the second spacer 610b are configured to receive the second spinous process SP2, After the first and second spacers 610a, 610b are positioned between the first and second spinous processes SP1, SP2, each elongate member from the set of elongate members 650 is slidably inserted between the first and second spacers 610a, 610b. In such embodiments, an insertion tool (discussed above) can be used to lift the proximal end portion of the first spacer 610a and/or the proximal end portion of the second spacer 610b to facilitate the insertion of the elongate members 650 between the first and second spacers 610a, 610b. Alternatively, the set of elongate members 650 can be inserted following the insertion of the first spacer 610a and prior to the insertion of the second spacer 610b.

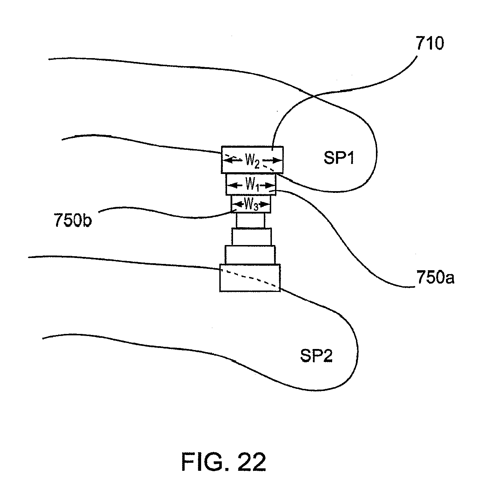

The elongate members 550 and 650 described herein can define a variety of shapes, sizes, and configurations. For example, in some embodiments, each elongate member can have a substantially rectangular cross-section. In other embodiments, a width of an elongate member from the set of elongate members can be different from a width of an adjacent elongate member or the spacer. For example, in some embodiments, as shown in FIG. 22, a width W₁ of the first elongate member 750a from the set of elongate members 750 in a direction substantially normal to the longitudinal axis L-L of the first elongate member 750a is less than a width W₂ of the spacer 710 in the direction. Moreover, a width W₃ of the second elongate member 750b in a direction substantially normal to the longitudinal axis L₁-L₁ of the second elongate member 750b is less than a width W₁ of the first elongate member 750a. Variable widths of the elongate members 750 can minimize opening trauma of the interspinous ligament. For purposes of clarity, the longitudinal axes L-L and L₁-L₁ are not illustrated in FIG. 22. Axes L-L and L₁-L₁ are illustrated, for example in FIG. 21.

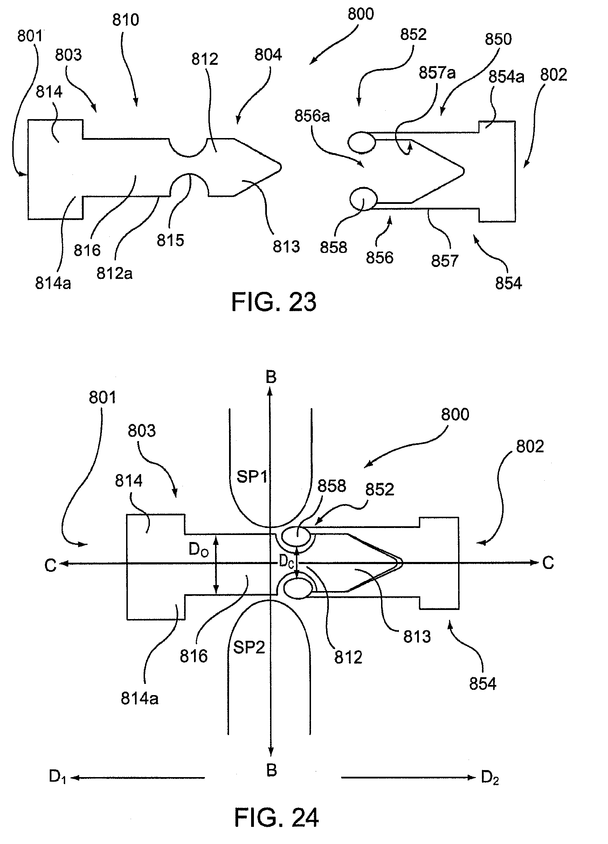

FIGS. 23 and 24 are schematic illustrations of an implant 800 according to another embodiment, and illustrate posterior cross-sectional views of the implant 800 disposed between a first spinous process SP1 and a second spinous process SP2 adjacent the first spinous process SP1. The implant 800 includes a spacer 810 and a retention member 850. When coupled, the spacer 810 and the retention member 850 collectively define a proximal end portion 801 of the implant 800 and a distal end portion 802 of the implant 800.

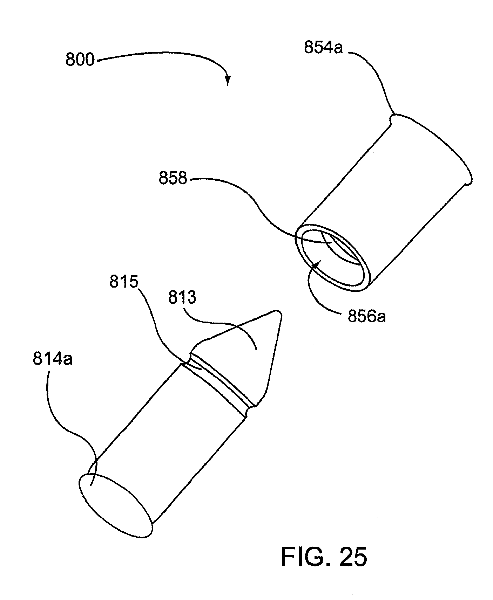

The spacer 810 has a proximal end portion 803 and a distal end portion 804. The spacer 810 includes first portion 814 disposed at its proximal end portion 803, a second portion disposed 812 at its distal end portion 804, and a third portion 816 extending between the first portion 814 and the second portion 812. The first portion 814 includes a flange 814a configured to limit movement of the implant 800 in a second lateral direction D₂ relative to the first spinous processes SP1 and the second spinous process SP2 when the spacer 810 is disposed between the spinous processes SP1, SP2. The second portion 812 includes a dilator portion 813 and defines an annular groove 815 in an outer surface 812a of the first portion 812 (shown in FIGS. 24 and 25). In some embodiments, a surface of the second portion is tapered from the annular groove to a distal end of the spacer. Said another way, in such embodiments, a diameter of the second portion continuously decreases from the annular groove to the distal end of the spacer, as shown in FIG. 23. The third portion 816 is configured to be disposed between the first spinous processes SP1 and the second spinous process SP2.

The retention member 850 includes a distal end portion 854 and a proximal end portion 852. The distal end portion 854 includes a flange 854a portion configured to limit lateral movement of the implant 800 relative to the spinous processes SP1, SP2 in a first direction D₁ opposite the second direction D₂ when the spacer 810 is disposed between the adjacent spinous processes SP1, SP2 and when the retention member 850 is coupled to the spacer 810. The proximal end portion 852 includes a coupling portion 856. The coupling portion 856 includes a sidewall 857 and defines an opening 856a configured to receive at least a portion of the dilator portion 813 of the second portion 812 of the spacer 810. The coupling portion 856 includes a protrusion 858 disposed along at least a portion of a circumference of an inner surface 857a of the side wall 857 of the coupling portion 856. The protrusion 858 is configured to be received within the annular groove 815 of the spacer 810 to releasably couple the retention member 850 to the spacer 810 as shown in FIG. 24.

An inner diameter D_{C} of the circumferential protrusion 858 of the coupling portion 856 is smaller than an outer diameter D_{O} of the outer surface of the distal end portion of the spacer 810, as shown in FIG. 24. This allows the circumferential protrusion 858 to fit snugly (e.g., interference fit, snap fit, etc.) within the annular groove 815 of the spacer 810.

The spacer 810 and the retention member 850, collectively, are configured to be disposed between the first and second spinous processes SP1, SP2 such that a longitudinal axis C-C defined by the spacer 810 and the retention member 850 is substantially orthogonal to a mid-line axis B-B defined by the first and second spinous process SP1, SP2. Said another way, the longitudinal axis C-C extends lengthwise (or longitudinally) (e.g., from the proximal end portion 801 to the distal end portion 802 of the implant 800) through the center of the spacer 810 and the retention member 850 and substantially perpendicular with respect to the mid-line axis B-B of the spinous processes SP1, SP2 when the implant is implanted.

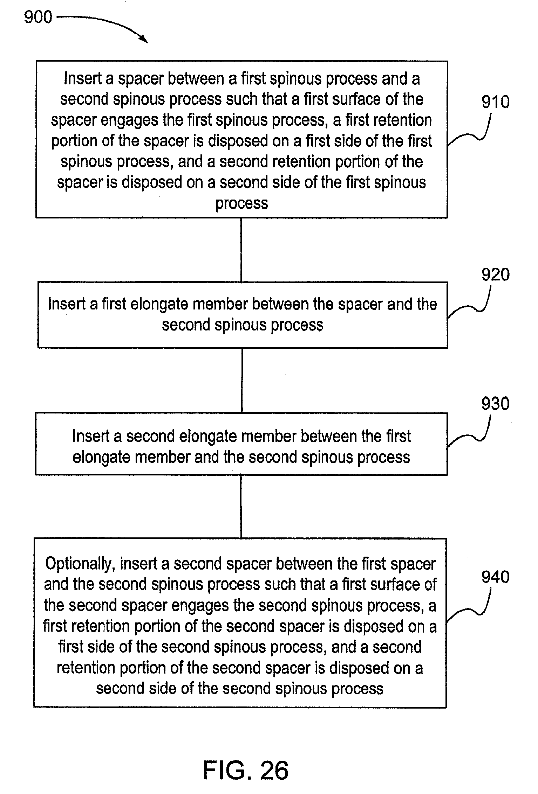

FIG. 26 is a flowchart of a method 900 for inserting an apparatus between a first spinous process and a second process according to an embodiment. At 910, a spacer of the implant is inserted between the first spinous process and the second spinous process such that a first surface of the spacer engages the first spinous process, a first retention portion of the spacer is disposed on a first side of the first spinous process, and a second retention portion of the spacer is disposed on a second side of the first spinous process. The second side is opposite the first side. In this manner, the first retention portion and the second retention portion collectively limit lateral movement of the spacer relative to the first spinous process.

At 920, a first elongate member from a set of elongate members is inserted between the spacer and the second spinous process such that the first elongate member slidably engages a second surface of the support portion of the spacer. Specifically, an upper surface of the first elongate member slidably engages a second surface of the spacer, as described above.

The first elongate member can be slidably engaged to the spacer by any suitable mechanism for providing various degrees of freedom. For example, in some embodiments the first elongate member and spacer are slidably coupled. For example, in some embodiments, the first elongate member can be magnetically coupled to spacer. In other embodiments, the first elongate member and the spacer can include complimentary projections and/or detents. Specifically, the upper surface of the first elongate member can include at least one projection. In such embodiments, the second surface of the support portion of the spacer includes at least one detent corresponding to the at least one projection. In yet other embodiments, for example, the upper surface of the first elongate member can include at least one ridge extending from the proximal end portion to the distal end portion of the first elongate member. In such embodiments, the lower surface of the support portion of the spacer includes at least one groove corresponding to the at least one ridge. In yet other embodiments, the second elongate member and the first elongate member can include mating protrusions and/or openings to releasably and slidably couple the second elongate member to the first elongate member.

At 930, a second elongate member from the set of elongate members is inserted between the first elongate member and the second spinous process such the second elongate member slidably engages the first elongate member. Specifically, an upper surface of the second elongate member slidably engages a lower surface of the first elongate member, as described above.

The second elongate member can be slidably engaged to the first elongate member by any suitable mechanism for providing various degrees of freedom. For example, in some embodiments, the upper surface of the second elongate spacer can be magnetically coupled to a lower surface of the first elongate member. In other embodiments, the upper surface of the second elongate member can include at least one ridge extending from the proximal end portion to the distal end portion of the second elongate member. In such embodiments, the lower surface of the first elongate member includes at least one groove corresponding to the at least one ridge.

Optionally, in some embodiments, at 940, a second spacer can be inserted, before inserting the elongate members. In such embodiments, the second spacer is inserted between the first spacer and the second spinous process, such that a first surface of the second spacer engages the second spinous process, a first retention portion of the second spacer is disposed on a first side of the second spinous process, and a second retention portion of the second spacer is disposed on a second side of the second spinous process. The second side of the second spinous process is opposite the first side of the second spinous process. In this manner, the first retention portion of the second spacer and the second retention portion of the second spacer collectively limit lateral movement of the space relative to the second spinous process

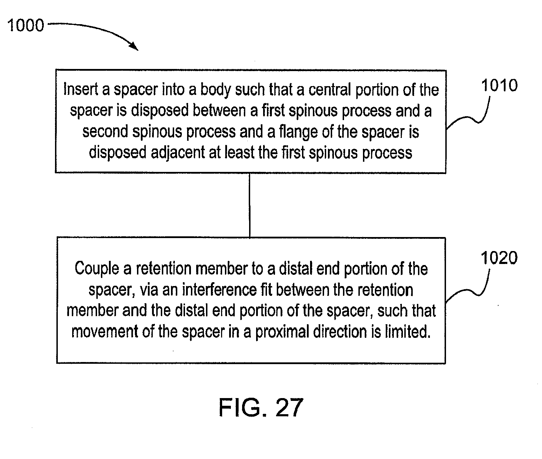

FIG. 27 is a flowchart of a method 1000 for inserting an apparatus between a first spinous process and a second process according to another embodiment. At 1010, a spacer is inserted into a body such that a central portion of the spacer is disposed between the first and second spinous processes, and a flange of the spacer is disposed adjacent at least the first spinous process. In this manner, lateral movement of the spacer relative to the first spinous process in a distal direction is limited. In some embodiments, the spacer can be inserted via a first lateral incision on a first side of the spine. Similarly stated, in some embodiments, the spacer can be inserted laterally from the first side of the spine.

At 1020, after the inserting of the spacer, a retention member is coupled to a distal end portion of the spacer, via an interference fit between the retention member and the distal end portion of the spacer, such that movement of the spacer in a proximal direction is limited. In some embodiments, for example, the retention member includes a flange to limit movement of the implant (i.e., spacer and retention member) in a proximal direction. In some embodiments, the retention member can be inserted via a second lateral incision on a second side of the spine opposite the first side of the spine. Similarly stated, in some embodiments, the retention member can be inserted laterally from the second side of the spine

The retention member and the spacer can be coupled by any suitable interference fit. For example, in some embodiments, the retention member receives a portion of the spacer such that the coupling is achieved via a mating groove and protrusion. In other embodiments, the retention member includes an annular protrusion and the spacer defines an annular groove, such that when the retention member is coupled to the spacer, the annular protrusion of the retention member mates with, or is received in, the annular groove of the spacer.

While various embodiments of the invention have been described above, it should be understood that they have been presented by way of example only, and not limitation. Thus, the breadth and scope of the invention should not be limited by any of the above-described embodiments, but should be defined only in accordance with the following claims and their equivalents. While the invention has been particularly shown and described with reference to specific embodiments thereof, it will be understood that various changes in form and details may be made.

The previous description of the embodiments is provided to enable any person skilled in the art to make or use the invention. While the invention has been particularly shown and described with reference to embodiments thereof, it will be understood by those skilled in art that various changes in form and details may be made. For example, a spinal implant can include various combinations and sub-combinations of the various embodiments described herein.

## Claims

1. An apparatus, comprising:
a spacer having a support portion, a first retention portion and a second retention portion, a first surface of the support portion configured to engage a first spinous process, the first retention portion configured to limit movement of the spacer relative to the first spinous process in a first lateral direction when the spacer is disposed between the first spinous process and a second spinous process, the second retention portion configured to limit movement of the spacer relative to the first spinous process in a second lateral direction opposite the first lateral direction when the spacer is disposed between the first spinous process and the second spinous process; and
a plurality of elongate members, a first elongate member from the plurality of elongate members slidably coupled to a second surface of the support portion of the spacer and a second elongate member from the plurality of elongate members.

2. The apparatus of claim 1, wherein:
the first elongate member from the plurality of elongate members is configured to be coupled to the second surface of the support portion of the spacer and the second elongate member from the plurality of elongate members.

3. The apparatus of either of claims 1 and 2 wherein:
the first elongate member from the plurality of elongate members is configured to be magnetically coupled to the second surface of the support portion of the spacer.

4. The apparatus of any preceding claim, wherein:
a width of the first elongate member from the plurality of elongate members in a direction substantially normal to the longitudinal axis of the first elongate member from the plurality of elongate members is less than a width of the spacer in the direction.

5. The apparatus of any preceding claim, wherein the spacer is a first spacer, the apparatus further comprising:
a second spacer having a support portion, a first retention of portion and a second retention portion, a first surface of the support portion of the second spacer configured to engage the second spinous process, the first retention portion of the second spacer configured to limit movement of the second spacer relative to the second spinous process in the first lateral direction when the second spacer is disposed between the first spinous process and the second spinous process, the second retention portion of the second spacer configured to limited movement of the second spacer relative to the second spinous process in the second lateral direction when the second spacer is disposed between the first spinous process and the second spinous process,
a third elongate member from the plurality of elongate members configured to slidingly engage a second surface of the support portion of the second spacer and a remaining elongate member from the plurality of elongate members.
